**Europäisches Patentamt**

**European Patent Office**

**Office européen des brevets**

(19)

(11) Veröffentlichungsnummer: **0 013 924**
**B1**

# (12) EUROPÄISCHE PATENTSCHRIFT

(45) Veröffentlichungstag der Patentschrift:
**25.11.81**

(51) Int. Cl.³: **C 07 C 43/20**, C 07 C 41/14,
C 07 C 43/23

(21) Anmeldenummer: **80100159.5**

(22) Anmeldetag: **14.01.80**

(54) **Verfahren zur Herstellung von Alkylarylethern.**

(30) Priorität: **26.01.79 DE 2903020**

(43) Veröffentlichungstag der Anmeldung:
**06.08.80 Patentblatt 80/16**

(45) Bekanntmachung des Hinweises auf die Patenterteilung:
**25.11.81 Patentblatt 81/47**

(84) Benannte Vertragsstaaten:
**BE CH DE FR GB IT NL**

(56) Entgegenhaltungen:
**Keine**

(73) Patentinhaber: **BAYER AG, Zentralbereich Patente,
Marken und Lizenzen, D-5090 Leverkusen 1, Bayerwerk
(DE)**

(72) Erfinder: **Neumann, Rainer, Dr.,
Bodelschwinghstrasse 16, D-4150 Krefeld (DE)**
Erfinder: **Schwarz, Hans-Helmut, Dr., Ratherstrasse 90,
D-4150 Krefeld (DE)**
Erfinder: **Nachtsheim, Dieter, Dr., Kronenpuetzchen 25,
D-4047 Dormagen 1 (DE)**

EP 0 013 924 B1

## Verfahren zur Herstellung von Alkylarylethern

Die Erfindung betrifft ein Verfahren zur Herstellung von Alkylarylethern durch Umsetzung von Phenolen mit aliphatischen Ethern in Gegenwart stark saurer Kationenaustauscher.

Verfahren zur Herstellung von Alkylarylethern sind in großer Zahl bekannt (z. B. Houben—Weyl, Methoden der organischen Chemie, Band VI/3, Seite 54 ff. [1965]). So die Veretherung von Phenolen mit aliphatischen Alkoholen in Gegenwart saurer Kationenaustauscher (s. Nippon Kaishi 8, 1513 [1974]; USSR-PS 197 613; DE-OS 2 655 826) und die Veretherung von Phenolen mit aliphatischen Ethern in Gegenwart wäßriger Metallsalzlösungen (AT-PS 251 561). Diese Verfahren weisen jedoch wesentliche Nachteile auf. So ist das aliphatische Ether als Alkylierungsmittel verwendende Verfahren mit erheblichen Korrosions-, Abwasser- und Trennproblemen belastet.

Von den in Gegenwart stark saurer Kationenaustauscher mit aliphatischen Alkoholen als Alkylierungsmittel arbeitenden Verfahren liefert nur das in der DE-OS 2 655 826 beschriebene Verfahren gute Ausbeuten an Alkylarylethern. Diese Ausbeuten werden aber vorzugsweise mit primären aliphatischen Alkoholen erreicht. Bei Verwendung sekundärer und tertiärer Alkohole nehmen die Ausbeuten an kernalkylierungsproduktfreien Alkylarylethern infolge zunehmender Kernalkylierung ab.

Es wurde nun überraschenderweise gefunden, daß man Arylalkylether mit nur geringem Anteil an kernalkylierten Verbindungen in technischem Maßstab, in wirtschaftlicher Weise und guten Ausbeuten durch Verethern von Phenolen mit Ethern aliphatischer Alkohole in Gegenwart stark saurer Kationenaustauscher herstellen kann.

Die Erfindung betrifft daher ein Verfahren zur Herstellung von Arylalkylethern durch Alkylierung von Phenolen in Gegenwart stark saurer Kationenaustauscher, das dadurch gekennzeichnet ist, daß man als Alkylierungsmittel Ether aliphatischer Alkohole verwendet.

Das erfindungsgemäße Verfahren eignet sich besonders zur Alkylierung von Phenolen mit sekundären und tertiären aliphatischen Alkylresten, vor allem zur Isopropylierung von Phenolen.

Das Molverhältnis Phenol zu aliphatischer Ether ist in dem erfindungsgemäßen Verfahren ohne Bedeutung. Da die als Alkylierungsmittel zu verwendenden aliphatischen Ether gute Lösungsmittel für die zu verethernden Phenole darstellen, wird man die Ether in einer solchen Menge einsetzen, daß eine bei der Reaktionstemperatur homogene Phenollösung erhalten wird.

Als Vertreter der erfindungsgemäß zu alkylierenden Phenole seien beispielsweise genannt:

Phenol, durch $C_1—C_4$-Alkylgruppen substituierte Phenole wie m-, o-, p-Kresol, Xylenole und tert.-Butylphenol, durch Halogenatome substituierte Phenole wie m-, o-, p-Chlorphenol und 2,4-Dichlorphenol, ferner Polyphenole wie Brenzkatechin, Resorcin, Hydrochinon und 1,2,4-Trihydroxybenzol, ferner $\alpha$- und $\beta$-Naphthol und Hydroxyanthracen.

Bei den in dem erfindungsgemäßen Verfahren als Alkylierungsmittel verwendeten aliphatischen Ethern handelt es sich um Ether deren aliphatische Reste 1 bis 8, vorzugsweise 1 bis 4, Kohlenstoffatome aufweisen. Beispielsweise seien genannt: Dimethylether, Diäthylether, Dipropylether, Diisopropylether, Diisobutylether und Di-tert.-butylether.

Einige dieser aliphatischen Ether fallen als Abfallprodukte bei der Herstellung der entsprechenden aliphatischen Alkohole an. Das erfindungsgemäße Verfahren ermöglicht eine sinnvolle Verwertung dieser Abfallprodukte.

In dem erfindungsgemäßen Verfahren sind grundsätzlich alle stark sauren Kationenaustauscher auf Kunstharzbasis verwendbar. Solche stark sauren Kationenaustauscher sind z. B. in Kirk—Othmer, Encyclopedia of Chemical Technology, 2nd Edition, Vol. 11, Seiten 871 und ff. beschrieben. Die stark sauren Kationenaustauscher können sowohl gelförmig wie auch makroporös sein. Die Kationenaustauscher werden in dem erfindungsgemäßen Verfahren in ihrer $H^+$-Form eingesetzt. Die Kationenaustauscher werden vorzugsweise in Form handelsüblicher Perlpolymerisate, aber auch in Form von Granulaten oder auch in Pulverform verwendet.

Die Menge der stark sauren Kationenaustauscher kann bei der Durchführung des erfindungsgemäßen Verfahrens in weiten Grenzen schwanken. Im allgemeinen wird der stark saure Kationenaustauscher in Mengen von 0,5 bis 50 Gew.-%, vorzugsweise 1 bis 25 Gew.-%, bezogen auf das Gewicht der Ausgangsverbindungen, eingesetzt.

Da die als Alkylierungsmittel zu verwendenden aliphatischen Ether gute Lösungsmittel für die zu verethernden Phenole darstellen, kann auf die Verwendung eines Lösungsmittels im allgemeinen verzichtet werden.

Das erfindungsgemäße Verfahren läßt sich bei Unter-, Normal- oder Überdruck durchführen. Zur Erzielung hoher Reaktionsgeschwindigkeiten und guter Raum/Zeit-Ausbeuten kann es vorteilhaft sein, unter erhöhtem Druck zu arbeiten. Dies gilt vor allem beim Einsatz von niedrig siedenden aliphatischen Ethern, die bei der Reaktionstemperatur gasförmig sind. In diesem Fall sind Drucke, die über dem Eigendampfdruck der am niedrigsten siedenden Komponente liegenden zweckmäßig.

Das erfindungsgemäße Verfahren kann z. B. in der Weise ausgeführt werden, daß man das zu verethernde Phenol in dem als Alkylierungsmittel dienenden Ether löst, der Lösung den stark sauren Kationenaustauscher zusetzt und das Gemisch auf Reaktionstemperaturen von 20 bis 150°C,

2

vorzugsweise 50 bis 130° C, erwärmt.

Das erfindungsgemäße Verfahren kann diskontinuierlich und kontinuierlich durchgeführt werden. Die kontinuierliche Arbeitsweise hat sich besonders bewährt. Zum Beispiel kann man das aus Phenol, aliphatischem Ether und Katalysator bestehende Reaktionsgemisch kontinuierlich in einem Reaktor umsetzen, in dem man das Gemisch an einem Ende des Reaktors einführt und nach einer gewissen Verweilzeit bei der gewünschten Reaktionstemperatur kontinuierlich aus dem Reaktor abführt. Als besonders vorteilhaft hat es sich erwiesen, den Katalysator in dem Reaktor in einem Festbett anzuordnen. In diesem Falle wird das Phenol-Ether-Gemisch in kontinuierlichem Strom durch den in einem Festbett angeordneten sauren Kationenaustauscher geführt.

Eine besonders vorteilhafte, diskontinuierlich oder kontinuierlich ausführbare Ausführungsform des erfindungsgemäßen Verfahrens, durch die die Bildung kernalkylierter Verbindungen wesentlich herabgesetzt, d. h. die Selektivität der Alkylierungsreaktion stark erhöht wird, besteht darin, nach beendeter Umsetzung den Alkylarylether und die unterhalb seines Siedepunktes siedenden Verbindungen, wie Wasser, Alkohol, Dialkylether aus dem Reaktionsgemisch abzudestillieren und den Destillationsrückstand, gegebenenfalls nach Ergänzen des durch die Alkylaryletherbildung verbrauchten Phenols und/oder Dialkylethers, erneut umzusetzen.

Das überraschende an dieser Arbeitsweise ist, daß durch das Wiedereinsetzen des Destillationsrückstandes der Anteil an kernalkylierten Verbindungen abnimmt und nicht, wie dies zu erwarten war, gleich bleibt.

Bei dieser Arbeitsweise kann es vorteilhaft sein, die Umsetzung nur bis zu einem gewissen Prozentsatz des theoretischen Umsatzes durchzuführen.

Die Aufarbeitung der bei dem erfindungsgemäßen Verfahren anfallenden Reaktionsgemische erfolgt nach an sich bekannten Verfahren: Zunächst wird der feste Katalysator, falls nicht in einem Festbett gearbeitet wurde, nach üblichen Methoden der Fest-Flüssig-Trennung, wie Filtrieren, Zentrifugieren, Sedimentieren und Dekantieren, entfernt. Der Katalysator kann für neue Umsetzungen wiederverwendet werden. Aus der flüssigen Phase werden zunächst bei der Umsetzung gebildetes Wasser, nicht umgesetzter aliphatischer Ether durch Destillation abgetrennt. Der verbleibende Rückstand enthält den entstandenen Alkylarylether, der nach an sich bekannten Verfahren, z. B. Destillieren oder Kristallisieren, isoliert wird.

Die nach dem erfindungsgemäßen Verfahren erhältlichen Alkylarylether sind Zwischenprodukte für die Herstellung von Schädlingsbekämpfungsmitteln, Farbstoffen und Hilfsprodukten für Kunststoffe (s. DE-AS 1 643 358), Riechstoffe, Antioxidantien, Konservierungsmittel (s. Ullmanns Encyklopädie der technischen Chemie, Band 13, Seite 450 [1962]).

Bei den in den folgenden Beispielen angegebenen Teilen handelt es sich, sofern nichts anderes angegeben ist, um Gewichtsteile.

### Beispiel 1

282 Teile Phenol und 56,4 Teile (= 20 Gew.-%) trockener, stark saurer Kationenaustauscher (mit 4 Gew.-% Divinylbenzol vernetzte Polystyrolsulfonsäure) werden in einem mit Rührer, Rückflußkühler, Thermometer und Gaseinleitungsrohr versehenen Dreihalskolben auf 120° C erhitzt. Dann werden innerhalb von 2¹/₂ Stunden 10 Teile Dimethylether in die Reaktionsmischung eingeleitet. Das Reaktionsgemisch hatte, wie gaschromatographisch bestimmt wurde, folgende Zusammensetzung: 251 Teile Phenol, 35,6 Teile Anisol, 3 Teile Wasser, 2,5 Teile Dimethylether. Der Anteil kernalkylierter Produkte lag unter 1% bezogen auf Anisol.

### Beispiel 2

In einem senkrecht stehenden Rohr (innerer Durchmesser: 40 mm; Rauminhalt: 2 l) werden 500 Teile trockener, stark saurer Kationenaustauscher (mit 4 Gew.-% Divinylbenzol vernetzter Polystyrolsulfonsäure) in einem Festbett angeordnet und mit Brenzkatechin bis zu Volumenkonstanz aufgequollen. Der Reaktor ist an seinem unteren Ende mit einer Zuführungsleitung versehen, durch die die Reaktionslösung mit einem den Druckverlust in der Säule ausgleichenden Gegendruck zudosiert wird. An seinem oberen Ende ist der Reaktor mit einem Abfluß versehen, durch den das Reaktionsgemisch in eine Vorlage abfließen kann.

Eine auf 110° C erwärmte Lösung aus 1100 Teilen Brenzkatechin und 79 Teilen Diäthylether (Molverhältnis 10 : 1) durchströmt das Festbett von unten nach oben. Die mittlere Verweilzeit der flüssigen Phase, bezogen auf das Leerrohr, beträgt 8 Stunden. Durch eine Außenheizung wird im Innern des Reaktors eine Temperatur von 120° C eingestellt.

Nachdem sich ein stationärer Zustand eingestellt hat, weist die am oberen Ende des Reaktors ablaufende Reaktionslösung folgende (gaschromatographisch bestimmte) Zusammensetzung auf: 984 Teile Brenzkatechin, 152 Teile o-Äthoxyphenol (Guäthol), 9 Teile Wasser, 36 Teile Diäthylether und 3 Teile unbekannte, wahrscheinlich kernalkylierte Verbindungen; d. h. die Ausbeute an (Guäthol, bezogen auf umgesetztes Brenzkatechin, beträgt 97%. Der Anteil an kernalkylierten Verbindungen,

**0 013 924**

bezogen auf Guäthol, liegt unter 2%, der Anteil an o-Diäthoxybenzol, bezogen auf Guäthol, liegt unter 0,7%.

Beispiel 3

Die auf 60° C vorgewärmte Lösung von Brenzkatechin in Diisopropylether (Molverhältnis 1 : 1) wird in den in Beispiel 2 beschriebenen Reaktor eingespeist. Die mittlere Verweilzeit der flüssigen Phase im Reaktor beträgt 10 Stunden. Durch eine Außenheizung wird im Innern des Reaktors eine Temperatur von 70° C eingestellt.

Nachdem sich ein stationärer Zustand eingestellt hat werden aus der aus dem Reaktor ablaufenden Reaktionslösung zunächst Diisopropylether und Wasser abdestilliert. Das von diesen beiden Verbindungen befreite Reaktionsgemisch wird gaschromatographisch analysiert. Es weist die in Tabelle 1, Spalte a) angegebene Zusammensetzung auf.

Anschließend wird aus dem Reaktionsgemisch das o-Isopropoxyphenol abdestilliert.

Der Destillationsrückstand wird dann mit einer der abdestillierten o-Isopropoxyphenol-Menge äquivalenten Menge Brenzkatechin versetzt. Die Mischung wird mit der gleichen Gewichtsmenge Diisopropylether verdünnt und erneut bei 70° C wie vorstehend beschrieben im Reaktor umgesetzt. Die ablaufende Reaktionslösung wird wiederum durch Destillation von Diisopropylether und Wasser und — nach ihrer gaschromatographischen Analyse — vom gebildeten o-Isopropoxyphenol befreit und der Rückstand wiederum nach Ergänzen des eingesetzten Brenzkatechins und Diisopropylethers erneut umgesetzt. Der Vorgang wurde insgesamt 5mal wiederholt. In der nachstehenden Tabelle 1 sind die Zusammensetzungen der Reaktionslösungen jeweils vor der Abtrennung des o-Isopropoxyphenols angegeben, ferner der bei jedem Durchlauf erreichte Umsatz und die bei jedem Durchlauf erreichte Selektivität.

Tabelle 1

| | Zusammensetzung (%) der nach Abdestillieren des Reaktionswassers und des Diisopropyläthers anfallenden Reaktionsgemische nach dem | | | | | |
|---|---|---|---|---|---|---|
| | a) 1. Durch- lauf | b) 2. Durch- lauf | c) 3. Durch- lauf | d) 4. Durch- lauf | e) 5. Durch- lauf | f) 6. Durch- lauf |
| ortho-Isopropoxyphenol | 28 | 24 | 24 | 19 | 16 | 16 |
| Brenzkatechin | 56 | 45 | 34 | 26 | 27 | 29 |
| unbek. Komponenten | 16 | 31 | 42 | 55 | 57 | 55 |
| Umsatz | 37% | 40% | 45% | 50% | 35% | 30% |
| Selektivität | 64% | 54% | 63% | 52% | 76% | 89% |

$$\text{Umsatz (\%)} = \frac{\text{umgesetztes Brenzkatechin (pro Kreislauf)}}{\text{eingesetztem Brenzkatechin (pro Kreislauf)}}$$

$$\text{Selektivität (\%)} = \frac{\text{zu ortho-Isopropoxyphenol umgesetztes Brenzkatechin (pro Kreislauf)}}{\text{gesamt umgesetztes Brenzkatechin (pro Kreislauf)}}$$

**Patentansprüche**

1. Verfahren zur Herstellung von Alkylarylethern durch Alkylieren von Phenolen in Gegenwart stark saurer Kationenaustauscher, dadurch gekennzeichnet, daß man als Alkylierungsmittel Dialkylether verwendet.

2. Verfahren gemäß Anspruch 1, dadurch gekennzeichnet, daß man als Alkylierungsmittel Dialkylether sekundärer oder tertiärer aliphatischer Alkohole verwendet.

3. Verfahren gemäß Anspruch 1 und 2, dadurch gekennzeichnet, daß man als Alkylierungsmittel Di-Isopropyläther verwendet.

4

**0 013 924**

4. Verfahren gemäß Anspruch 1 bis 3, dadurch gekennzeichnet, daß man nach beendeter Umsetzung den Alkylarylether und die unterhalb seines Siedepunktes siedenden Verbindungen aus dem Reaktionsgemisch abdestilliert und den Destillationsrückstand, gegebenenfalls nach Ergänzen des durch die Alkylaryletherbildung verbrauchten Phenols und/oder Dialkylethers, erneut umsetzt.

## Claims

1. Process for the preparation of alkyl aryl ethers by alkylating phenols in the presence of strongly acid cation exchangers, characterised in that dialkyl ethers are used as the alkylating agent.

2. Process according to claim 1, characterised in that dialkyl ethers of secondary or tertiary aliphatic alcohols are used as the alkylating agent.

3. Process according to claim 1 and 2, characterised in that di-isopropyl ether is used as the alkylating agent.

4. Process according to Claim 1 to 3, characterised in that, when the reaction has ended, the alkyl aryl ether and the compounds which have lower boiling points than the latter are distilled off from the reaction mixture and the distillation residue is reacted again, if appropriate after replacing the phenol and/or dialkyl ether consumed by the alkyl aryl ether formation.

## Revendications

1. Procédé pour la préparation d'alkylaryléthers par alkylation de phénols en présence d'échangeurs de cations fortement acides, caractérisé en ce qu'on utilise comme agents d'alkylation des éthers d'alcools aliphatiques.

2. Procédé selon la revendication 1, caractérisé en ce que l'on utilise comme agents d'alkylation des éthers dialkyliques d'alcools aliphatiques secondaires ou tertiaires.

3. Procédé selon les revendications 1 et 2, caractérisé en ce que l'on utilise comme agent d'alkylation l'éther diisopropylique.

4. Procédé selon l'une quelconque des revendications 1 à 3, caractérisé en ce que l'on élimine du mélange de réaction, par distillation, après la fin de la réaction, l'alkylaryléther et les composés bouillant au-dessous de son point d'ébullition, tels qu'alcool, dialkyléther, etc., et on fait réagir à nouveau le résidu de distillation, éventuellement après remplacement du phénol et/ou du dialkyléther consommé par la formation d'alkylaryléther.